**Europäisches Patentamt**

**European Patent Office**

**Office européen des brevets**

(19)

(11) **EP 1 031 836 A1**

(12) **DEMANDE DE BREVET EUROPEEN**

(43) Date de publication:
**30.08.2000 Bulletin 2000/35**

(51) Int Cl.⁷: **G01N 33/543**

(21) Numéro de dépôt: **00400449.5**

(22) Date de dépôt: **18.02.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **24.02.1999 FR 9902288**

(71) Demandeurs:
• **COMMISSARIAT A L'ENERGIE ATOMIQUE**
  **75752 Paris Cédex 15 (FR)**
• **BIOMERIEUX SA**
  **F-69280 Marcy L'Etoile (FR)**

(72) Inventeurs:
• **Fouillet, Yves**
  **38340 Voreppe (FR)**
• **Theretz, Alain**
  **69130 Ecully (FR)**
• **Perrin, Agnès**
  **69007 Lyon (FR)**

(74) Mandataire: **Des Termes, Monique et al**
**Société Brevatome**
**3, rue du Docteur Lancereaux**
**75008 Paris (FR)**

(54) **Procédé de détermination d'un analyte présent dans une solution**

(57)    L'invention concerne un procédé de détermination d'un analyte dans une solution.
    Ce procédé comprend les étapes suivantes :

    a) fixer et immobiliser ledit analyte, par exemple grâce à la présence d'un ligand L, sur la surface interne d'un conduit (3) ayant une section transversale réduite sur tout ou partie (9) de celui-ci, et
    b) déterminer la variation de la perte de charge d'un fluide circulant dans le conduit, due à l'analyte P qui a été fixé et immobilisé au moins dans la partie (9) de section transversale réduite dudit conduit lors de l'étape a).

FIG. 6

## Description

### Domaine technique

La présente invention a pour objet un procédé de détermination d'un analyte présent dans une solution. De façon plus précise, elle concerne un procédé permettant de détecter et/ou de quantifier cet analyte. L'analyte peut être une entité chimique ou biologique.

Aussi, la présente invention trouve de nombreuses applications dans le domaine de l'analyse chimique ou biologique, par exemple pour la détermination d'antigènes, d'anticorps, de séquences d'ADN ou d'ARN, de bactéries, de virus, de fragments de bactéries, etc...

### État de la technique antérieure

De nombreux systèmes d'analyses chimiques ou biologiques sont basés sur la réaction de l'analyte à déterminer avec un composé approprié pour former un produit de réaction qu'il convient ensuite de détecter et/ou de quantifier pour déterminer la présence et la concentration de cet analyte. Généralement, cette détection est effectuée par l'intermédiaire de marqueurs tels que des marqueurs radioactifs ou luminescents, des enzymes ou autres, qui peuvent être portés par le composé mis à réagir avec l'analyte à détecter. La mise en oeuvre de tels marqueurs suppose l'emploi d'appareillages spécifiques pour assurer la détection et/ou la quantification de l'analyte.

D'autres méthodes de détection et de quantification telles que des méthodes topographiques, par exemple par microscopie à force atomique (AFM), des méthodes magnétiques, des méthodes électriques, par exemple mesure d'une variation de capacité, et des méthodes optiques, peuvent aussi être utilisés comme il est décrit dans FR-A-2 758 884 [1].

Cependant, ces méthodes ont également l'inconvénient d'exiger des appareillages spécifiques qui sont parfois d'un coût élevé.

La présente invention a précisément pour objet un procédé de détermination d'un analyte qui évite l'emploi de tels appareillages, en assurant la détection et/ou la quantification de l'analyte au moyen de composants très classiques, d'un coût abordable.

### Exposé de l'invention

Selon l'invention, le procédé de détermination d'un analyte dans une solution comprend les étapes suivantes :

a) fixer et immobiliser ledit analyte sur la surface interne d'un conduit ayant une section transversale réduite sur tout ou partie de celui-ci, et

b) déterminer la variation de la perte de charge d'un fluide circulant dans le conduit, due à l'analyte qui a été fixé et immobilisé au moins dans la section transversale réduite dudit conduit lors de l'étape a).

Le procédé de l'invention est donc basé sur l'utilisation d'un conduit ayant une section transversale appropriée sur tout ou partie de celui-ci, dans lequel, dans l'étape a), on fixe et on immobilise l'analyte à déterminer.

La fixation et l'immobilisation de l'analyte se fait au moins sur toute ou partie de la section réduite du conduit mais elles peuvent avoir lieu également sur l'ensemble du conduit.

Cette fixation et cette immobilisation peuvent se faire soit par une mise en contact de la solution avec la surface interne du conduit (par exemple par trempage), soit par une circulation de la solution dans le conduit, cette circulation pouvant comporter au moins un arrêt séquentiel.

Dans l'étape b) qui suit, on détermine la variation de perte de charge d'un fluide circulant dans le conduit, variation qui résulte de la fixation de l'analyte et dépend de la quantité d'analyte fixé.

Cette quantification de l'analyte peut être effectuée à partir de courbes ou d'abaques préalablement déterminées, soit par expérimentation, soit par simulation, à partir des connaissances géométriques du conduit et des analytes utilisés (dimensions et quantités). Ainsi, la valeur de la perte de charge est fonction de la quantité d'analyte immobilisé dans le conduit et/ou de la taille de cet analyte.

Par expérimentation, on peut croiser la mesure obtenue dans le procédé de l'invention avec de mesures effectuées précédemment pour étalonner le comportement du conduit.

Ces deux méthodes permettent d'établir des abaques donnant la relation entre la perte de charge et la quantité d'analyte immobilisé. Ainsi, par une simple mesure de la perte de charge, on pourra quantifier le taux d'analyte immobilisé dans le conduit dans l'étape a) du procédé de l'invention.

Selon l'invention, on peut déterminer la variation de la perte de charge en mesurant la différence de pression à débit constant entre deux points situés de part et d'autre de la section transversale réduite du conduit, d'un fluide circulant dans le conduit, avant de réaliser l'étape a) et après avoir réalisé l'étape a) du procédé, ou la différence de débit à pression constante avant et après l'étape a).

Le fluide utilisé pour cette détermination peut être toute ou partie de la solution de départ (dans ce cas, l'étape a) et l'étape b) sont simultanées ou successives) ou un liquide différent de cette solution.

Dans l'art antérieur, on a utilisé la mesure de la perte de charge dans un conduit où circule un fluide pour déterminer certaines caractéristiques du fluide, par exemple sa viscosité, et en conséquence sa teneur en un produit particulier capable de modifier sa viscosité. Ainsi, dans le document US-A-4 964 847 [2], on utilise cette mesure pour estimer le taux d'hématocrite du sang.

Un tel procédé de détermination est différent du procédé de l'invention dans lequel la détection et la quantification de l'analyte nécessitent l'immobilisation de celui-ci sur la paroi d'un conduit de forme adaptée, ayant de plus la propriété de pouvoir fixer l'analyte à déterminer.

Cette dernière propriété peut être conférée au conduit en fixant sur sa paroi un ligand capable de se lier à l'analyte.

Dans ce cas, la détermination de l'analyte met en jeu une réaction de reconnaissance entre l'analyte et le ligand avec formation d'un complexe analyte-ligand immobilisé dans le conduit.

Dans ce mode de réalisation du procédé de l'invention, préalablement à l'étape a), la paroi de la section transversale réduite est revêtue sur tout ou partie d'au moins un ligand capable de se fixer à l'analyte.

Dans ce cas, on peut détecter la présence du ligand en déterminant la variation de la perte de charge en mesurant la différence de pression à débit constant ou la différence de débit à pression constante entre deux points situés de part et d'autre de la section transversale réduite du conduit, d'un liquide circulant dans le conduit, avant et après avoir réalisé le revêtement de ligand.

Selon une variante de réalisation de l'invention, lorsque l'analyte à déterminer ou le complexe formé par réaction de cet analyte avec un ligand a une dimension trop faible pour engendrer une perte de charge suffisante dans la portion de section transversale réduite du conduit, on effectue de plus une réaction physique ou chimique de l'analyte avec un matériau-support pour former un conjugué analyte-support de dimension plus importante que celle de l'analyte, cette réaction intervenant soit lorsque ledit analyte est libre dans la solution, avant l'étape a), soit lorsque l'analyte est déjà fixé directement ou indirectement sur la paroi interne de la section réduite du conduit après l'étape a) pour constituer un conjugué analyte-support.

Cette réaction entre l'analyte et le matériau-support peut être effectuée avant l'introduction de la solution dans le conduit, soit avant de réaliser l'étape a), ou après fixation de l'analyte sur la paroi de la portion de section transversale réduite du conduit, c'est-à-dire après l'étape a) mais avant l'étape b).

Pour favoriser la liaison du matériau-support avec l'analyte, celui-ci comprend généralement en surface un ligand capable de se lier à l'analyte.

Selon l'invention, on entend par « analyte » toute entité chimique ou biologique, notamment toute entité biologique sous forme libre. A titre d'exemple d'analytes, on peut citer les cellules, les organelles, les virus et les bactéries, les anticorps, les fragments d'anticorps, les antigènes, les haptènes, les lectines, les sucres, les acides ribonucléiques et désoxyribonucléiques, les protéines, notamment A ou G, les hormones, les récepteurs d'hormones, la biotine, l'avidine, la streptavidine, et d'une manière générale toute molécule ou macromolécule, naturelle ou synthétique, ou analogue, ou encore résultant d'une association d'au moins deux molécules ou macromolécules du type de celles définies précédemment. A titre d'exemple d'associations de molécules ou macromolécules, on peut citer les conjugués analytes-supports définis ultérieurement.

Selon l'invention, on entend par « matériau-support », tout type de support biologique, polymère, organique, inorganique ou métallique, qui peut être distribué ou dispersé sous forme discrète au sein d'un milieu liquide, et le plus souvent, mais sans que cela soit limitatif, sous forme de particules.

A titre d'exemple de matériau-support de type polymère, on peut citer les particules obtenues par polymérisation en émulsion telles que les particules de latex, ou des particules de plus grosses tailles ainsi que des polymères, copolymères et polymères greffés obtenus par d'autres moyens connus par l'homme de l'art.

A titre d'exemple de matériau-support de type métallique, on peut citer l'or colloïdal, les particules ferromagnétiques, ferrimagnétiques, paramagnétiques ou superparamagnétiques, recouvertes ou non de polymères naturels ou synthétiques, qui comprennent du fer ou d'autres métaux comme le cobalt, le nickel ou autres, seul ou sous forme d'alliages, magnétiques ou non.

A titre d'exemple de matériau-support de type inorganique, on peut citer les particules à base de silice ou de silicium, de mica, de verre et/ou de quartz.

A titre d'exemple de matériau-support de type biologique, on peut citer les protéines de masse ou de dimensions supérieures à celles de l'analyte à déterminer à l'état libre.

Par « conjugué analyte support », on entend tout analyte tel que défini précédemment, immobilisé sur un matériau-support tel que défini précédemment par un moyen quelconque. L'immobilisation des analytes sur le matériau-support peut s'effectuer soit par simple adsorption, soit par l'intermédiaire d'une réaction chimique ou physique permettant de modifier la surface du matériau-support et ainsi de permettre la fixation de l'analyte par des liens de covalence, soit par chélation, soit par reconnaissance moléculaire par l'intermédiaire d'un ligand immobilisé sur le matériau-support, et par tout autre moyen traditionnel bien connu de l'homme de l'art et capable de retenir un analyte.

On entend par « ligand », un élément capable de former, par un lien chimique ou physique, un complexe avec un analyte.

A titre d'exemple de ligand, on peut citer les anticorps, les fragments d'anticorps, les antigènes, les haptènes, les lectines, les sucres, les acides ribonucléiques, les acides désoxyribonucléiques, les protéines notamment A ou G, les hormones, les récepteurs d'hormones, la biotine, l'avidine ou la streptavidine et, d'une manière générale, les ligands naturels ou synthétiques et les analogues de ligands modifiés, pouvant entrer en compétition avec les ligands.

Dans la suite du présent texte, on désignera par « cellule à circulation » tout type de dispositif comportant le conduit avec sa portion de section transversale réduite, et au moins deux conduites d'entrée et de sortie permettant le passage d'un fluide dans le conduit.

On entend par « section transversale réduite », une section qui est généralement inférieure à celle du reste du conduit. Dans certains cas, l'ensemble du conduit peut avoir cette section réduite. Cette portion de section réduite peut être aussi appelée « restriction » et du fait de ses propriétés particulières de fixation de l'analyte, on la dénommera ci-après « zone active ».

Cette zone active est constituée ainsi par la paroi du conduit sur laquelle peuvent être fixés des ligands capables de se coupler à l'analyte.

Ces ligands sont fixés sur la paroi par un moyen quelconque tel que réaction chimique (liaison de covalence) ou physique (adsorption) pour modifier la surface de la paroi et permettre la fixation du ligand.

Selon l'invention, la section transversale réduite de la portion du conduit capable de fixer et immobiliser l'analyte, peut être de différentes formes. Elle peut être en particulier circulaire, le conduit étant de forme cylindrique, ou rectangulaire, l'écoulement étant dans ce cas un écoulement plan entre deux surfaces parallèles.

Le conduit peut être réalisé en divers matériaux, par exemple des matériaux de nature polymère, inorganique ou métallique.

A titre d'exemple de matériaux polymères, on peut citer les polymères à base de polystyrène, les polyacrylates, les polyméthacrylates, les polybutadiènes, les polypropylènes, ou d'autres seuls ou sous forme de copolymères.

A titre d'exemple de matériaux inorganiques, on peut citer l'oxyde de silicium, le nitrure de silicium, le silicium, le verre et le quartz.

Selon l'invention, la portion de section transversale réduite du conduit doit avoir des dimensions compatibles avec les dimensions de l'analyte à déterminer.

En effet, si les dimensions du conduit sont importantes par rapport à la taille de l'analyte immobilisé, la présence de cet analyte ne conduira pas à une variation significative de la perte de charge. On ne pourra donc ni détecter, ni quantifier l'analyte.

Les théories de la mécanique des fluides montrent que de la manière générale, la perte de charge $\Delta P$ entre l'entrée et la sortie d'une portion de conduit de section réduite est définie par la relation suivante :

$$\Delta P = KQ$$

avec Q représentant le débit volumique du liquide et K étant un coefficient de perte de charge qui dépend des caractéristiques géométriques du conduit et de la viscosité du liquide.

Suivant leurs dimensions respectives, la présence d'analytes ou de conjugués analytes-supports sur les parois du conduit entraîne une modification du coefficient de perte de charge. Avec une hauteur du conduit proche des dimensions de l'analyte ou du conjugué analyte-support, on obtient une variation du coefficient de perte de charge mesurable.

Aussi, pour effectuer la détermination de l'analyte dans de bonnes conditions, on utilise un conduit dont la portion de section transversale réduite a une dimension adaptée à la taille de l'analyte immobilisé ou du conjugué analyte-support immobilisé.

De préférence, la plus petite dimension $T_c$ de la section transversale réduite du conduit est telle que :

$$(T_c : 3) < T_A \text{ ou } e < T_c$$

avec $T_A$ représentant la dimension de l'analyte fixé tel quel ou sous la forme de conjugué analyte-support, sur la paroi de la portion de section transversale réduite du conduit, ou e représentant l'épaisseur du ligand.

Dans le cas d'un écoulement entre deux surfaces parallèles, $T_c$ représente l'écartement entre les deux surfaces. Dans le cas d'un conduit cylindrique, $T_c$ représente le diamètre du conduit.

Les dimensions des analytes ou des conjugués analytes-supports sont très faibles, puisque l'analyte peut être constituée aussi bien par une molécules que par une cellule vivante ; il est nécessaire d'avoir des conduits de petites dimensions, par exemple, supérieures à quelques centaines de nanomètres.

Des conduits présentant de telles dimensions peuvent être obtenus par les procédés de microfabrication tels que ceux mis en oeuvre en microélectronique, micromécanique et par exemple par un procédé du type de celui décrit dans FR-A-2 727 648 [3].

Le procédé de l'invention peut être mis en oeuvre de diverses façons, en effectuant de plus, si nécessaire, au moins une étape de lavage du conduit entre les étapes a) et b).

Selon un premier mode de réalisation qui peut être utilisé pour déterminer des analytes suffisamment volumineux, par exemple des bactéries, des virus ou des fragments de bactéries, le procédé comprend les étapes suivantes :

1) mettre en contact la solution contenant l'analyte avec la zone active du conduit pour fixer l'analyte sur cette zone active,
2) laver le conduit pour éliminer ce qui n'a pas réagi sur cette zone active, et
3) mesurer la perte de charge dans le conduit.

Selon un second mode de réalisation de l'invention, destiné à la détermination d'analytes de faible dimension, le procédé comprend les étapes suivantes :

1) réaction du matériau-support avec l'analyte dans la solution de départ, pour former le conjugué analyte-support dans cette solution,
2) mise en contact de la solution avec la zone active du conduit pour fixer le conjugué analyte-support sur cette zone active,
3) lavage du conduit pour éliminer ce qui n'a pas réagi, et,
4) mesure de la perte de charge dans le conduit.

Selon un troisième mode de réalisation de l'invention, destiné également à la détermination d'analytes de faible dimension, le procédé comprend les étapes suivantes :

1) mise en contact de la solution contenant l'analyte avec la zone active du conduit pour fixer l'analyte sur cette zone active,
2) lavage du conduit pour éliminer ce qui n'a pas réagi,
3) introduction dans la zone active du conduit d'une solution contenant un matériau-support pour former directement sur la zone active du conduit le conjugué analyte-support,
4) lavage du conduit pour éliminer le matériau-support qui n'a pas réagi, et
5) mesure de la perte de charge dans le conduit.

Bien qu'il soit préférable d'utiliser un liquide qui ne contienne aucune grosse molécule ou cellule biologique pour mesurer la perte de charge, on peut malgré tout envisager d'utiliser les liquides précédemment cités.

Ainsi, selon une variante de ces trois modes de réalisation, on supprime l'étape de lavage qui précède la mesure de la perte de charge, et on effectue cette mesure en utilisant comme fluide la solution introduite dans l'étape précédente, c'est-à-dire la solution d'analyte dans le premier mode de réalisation, la solution de conjugué analyte-support dans le deuxième mode de réalisation, et la solution de matériau-support dans le troisième mode de réalisation.

Selon l'invention, on peut aussi utiliser le même principe de base, mesure de la perte de charge induite par un produit immobilisé dans un conduit, pour déterminer la présence d'un revêtement de produit chimique ou biologique sur la surface interne d'un conduit.

Aussi, l'invention a encore pour objet un procédé pour déterminer la présence d'un revêtement de produit chimique ou biologique sur la surface interne d'un conduit, comprenant les étapes suivantes :

1) mettre un fluide en circulation dans le conduit,
2) mesurer la perte de charge dudit fluide dans le conduit, et
3) déterminer à partir de la perte de charge mesurée la présence ou non d'un revêtement dans le conduit.

Ce procédé peut être utile en particulier pour vérifier s'il y a bien un ligand fixé et immobilisé dans la zone active d'un conduit destiné à la détermination d'un analyte par mise en oeuvre du procédé de l'invention.

Dans ce dernier cas, il est bien entendu également nécessaire que les dimensions du conduit soient appropriées pour pouvoir déterminer la perte de charge induite par le revêtement.

De préférence, la plus petite dimension $T_c$ de la section du conduit est telle que :

$$(T_c : 3) < e < T_c$$

avec e représentant l'épaisseur du revêtement.

D'autres caractéristiques et avantages de l'invention apparaîtront mieux à la lecture de la description qui suit, donnée bien entendu à titre illustratif et non limitatif, en référence aux dessins annexés.

**Brève description des dessins**

La figure 1 est une représentation en coupe verticale et en perspective d'une cellule à circulation utilisée pour mettre en oeuvre le procédé de l'invention.

La figure 2 représente de façon schématique une installation comportant la cellule de la figure 1 pour mettre en oeuvre le procédé de l'invention et effectuer la mesure de perte de charge.

La figure 3 représente en coupe verticale, la zone active de la cellule de la figure 1 munie d'un revêtement de ligand.

La figure 4 est un diagramme illustrant la fixation d'un anticorps de capture sur la zone active de la cellule de la figure 1.

La figure 5 est un diagramme illustrant l'évolution de la perte de charge ΔP dans un conduit (en MPa) en fonction du débit Q dans le conduit (en ml/min).

La figure 6 représente en coupe verticale la zone active de la cellule de la figure 1, après immobilisation de l'analyte sur le ligand.

La figure 7 est un diagramme illustrant l'évolution de la perte de charge ΔP (en MPa) dans la zone active de la cellule en fonction des substances fixées.

La figure 8 représente en coupe verticale la cellule munie d'un revêtement de ligand L sur tout le conduit.

La figure 9 représente en coupe verticale la cellule de la figure 8, après immobilisation d'un conjugué analyte-support sur le ligand L.

**Exposé détaillé des modes de réalisation**

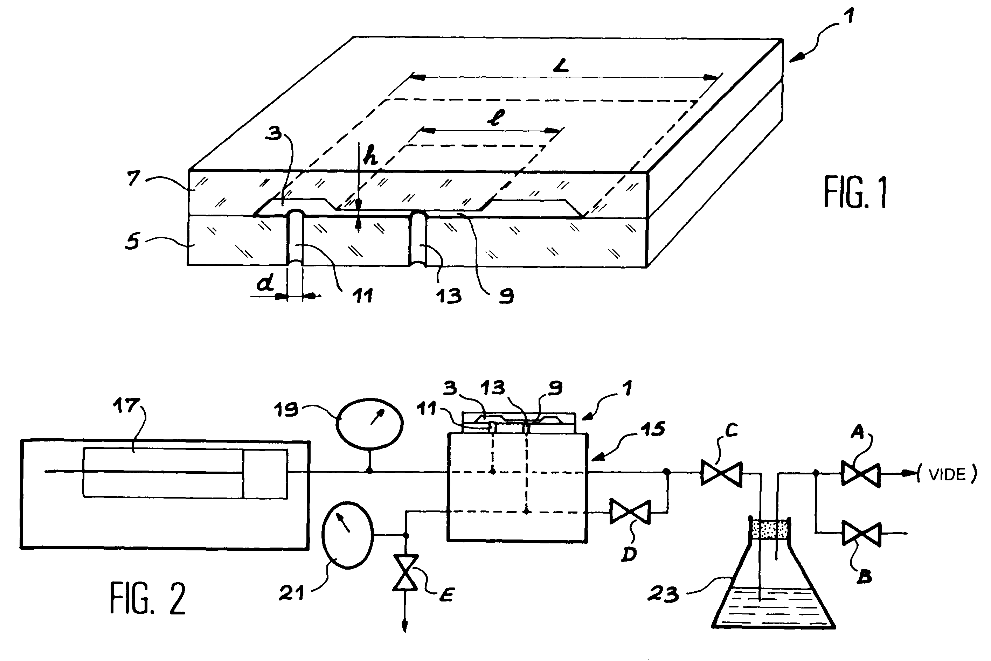

Sur la figure 1, on a représenté en coupe verticale et en perspective, une cellule pour la mise en circulation de la solution comportant l'analyte à déterminer.

Cette cellule 1 définit un conduit de circulation 3, entre une plaque de base 5 et une plaque supérieure 7 de la cellule qui sont parallèles, les plaques 5 et 7 étant usinées de façon à former le conduit de circulation 3 et la portion de section transversale réduite 9 qui est formée par une zone d'écoulement confiné entre les deux plans parallèles formés par les plaques 5 et 7. Cette zone d'écoulement 9 constitue la zone active de la cellule. La plaque de base 5 comporte deux orifices d'entrée 11 et de sortie 13 permettant de faire circuler une solution dans la cellule en passant dans la zone d'écoulement confiné 9.

A titre d'exemple, cette cellule peut présenter les dimensions suivantes : une hauteur h de 1 μm dans la zone d'écoulement confiné 9, une longueur l de la zone d'écoulement confiné 9 de 2 mm, un diamètre des orifices 9 et 11 d de 500 μm et une longueur L du conduit 3 de 5 mm. Cette cellule peut être réalisée par des procédés de microtechnologie à partir de deux substrats 5 et 7 en silicium. Dans le substrat inférieur 5, on peut usiner les orifices 9 et 11 par des procédés de gravure laser, gravure par plasma ou autres. Dans le substrat supérieur 7, on peut délimiter la zone d'écoulement confiné par des procédés de gravure.

On peut assembler ensuite les deux substrats par un procédé classique de scellement direct Si-Si.

Sur la figure 2, on a représenté un ensemble permettant d'effectuer les mesures nécessaires et la circulation des solutions dans la cellule de la figure 1.

Dans ce cas, la cellule 1 de la figure 1 est associée à un porte cellule 15, un pousse-seringue 17, un réseau de conduites munies des vannes A, B, C, D, et E, un ou deux capteurs de pression 19 et 21, et un flacon 23 comportant la solution à tester, une solution de rinçage ou un fluide pour mesurer la perte de charge. Les conduites munies de vannes permettent de mettre l'ensemble sous vide (vanne A) ou à la pression atmosphérique (vanne B).

Dans cette installation, la cellule 1 est collée au porte-cellule 15 ou fixée sur celui-ci par un système de serrage. Dans les deux cas, on assure un montage parfaitement étanche. Le pousse-seringue 17 permet d'imposer un débit contrôlé dans la cellule et de prélever les solutions provenant du flacon 23. La mesure de la perte de charge est effectuée grâce aux deux capteurs de pression 19 et 21 reliés respectivement à l'entrée 11 et à la sortie 13 de la cellule à circulation 1. Le second capteur de pression 21 n'est pas nécessaire quand le porte-cellule débouche à une pression de référence telle que la pression atmosphérique.

Pour avoir une mesure précise, il est nécessaire que le système soit entièrement rempli de liquide. En effet, les sections hydrauliques dans la cellule sont très petites et des bulles de gaz peuvent générer des forces de capillarité très importantes risquant de perturber la mesure de la perte de charge. Pour éliminer la formation de bulles qui restent prisonnières de la chambre de mesure, on peut dégazer les solutions mises en circulation dans celle-ci.

On peut arriver au même résultat avec l'installation représentée sur la figure 2 en mettant sous vide l'ensemble avant le premier remplissage et en dégazant les solutions avant manipulation.

Pour mettre en oeuvre le procédé de l'invention, il est nécessaire de faire circuler dans la cellule différentes solutions telles que des solutions d'analyte à déterminer et des solutions de rinçage. La forme du porte-cellule et les vannes C, D et E permettent de purger facilement l'ensemble des conduites à chaque changement de solution, tout en limitant l'apparition de bulles d'air dans l'installation.

Dans le tableau suivant, on a décrit un exemple de mode opératoire pour utiliser l'installation représentée sur la figure 2.

Tableau

| Phase | Description | Vannes (O : ouverte ; F : fermée) | | | | |
|-------|-------------|---|---|---|---|---|
| **Injection de la première solution** | | A | B | C | D | E |
| Phase 1 | Mise sous vide de l'ensemble | O | F | O | O | F |
| Phase 2 | Remplissage du circuit hydraulique et de la cellule | F | O | O | O | F |
| Phase 3 | Aspiration d'une quantité de solution dans la seringue | F | O | O | O | F |
| Phase 4 | Circulation de la solution dans la cellule | F | O | F | F | O |
| **Injection de la solution suivante** | | | | | | |
| Phase 5 | Changement de la solution (ou changement du flacon) | O | F | F | F | O |
| Phase 6 | Réitération des phases 3 et 4 | | | | | |

Dans la phase 1 de ce mode opératoire, on met sous vide l'ensemble de la cellule et des conduites, et on dégaze la solution du flacon 23 en ouvrant les vannes A, C et D et en fermant les vannes B et E.

Après cette opération, la phase 2 consiste à remplir le circuit hydraulique et la cellule en fermant les vannes A et E et en ouvrant les vannes B, C et D.

Dans la phase 3, on aspire une quantité de solution dans la seringue 17, en actionnant la seringue, les vannes B, C et D étant ouvertes et les vannes A et E étant fermées.

Dans la phase 4 qui suit, on fait circuler la solution dans la cellule en poussant la seringue 17 et en ouvrant les vannes B et E, les vannes A, C et D étant fermées. On peut alors mesurer la perte de charge dans la cellule au moyen du capteur de pression 19. Pour effectuer une nouvelle analyse ou un rinçage de l'ensemble, on peut changer le flacon 23, les vannes A et E étant ouvertes, et les vannes B, C et D étant fermées. On peut ensuite effectuer une nouvelle analyse en suivant le mode opératoire des phases 3 et 4 précédentes.

Dans cette cellule, la zone active qui correspond à la zone d'écoulement confiné 9, a été traitée pour que sa paroi soit apte à fixer un analyte ou un ligand de l'analyte à détecter.

Les exemples donnés ci-après illustrent, les traitements mis en oeuvre pour fixer sur la paroi en silicium de la zone active de la cellule un ligand constitué par des anticorps de capture, et l'utilisation de cette cellule pour déterminer un analyte constitué par d'autres anticorps capables d'être immobilisés par les anticorps de capture.

**Exemple 1 : Fonctionnalisation de la paroi de la zone d'écoulement confiné.**

Cette opération peut être effectuée sur les substrats 5 et 7 avant leur assemblage pour former la cellule.

Dans ce but, on ébouillante les structures dans l'eau pure pendant 2 heures, puis on les sèche sous atmosphère inerte d'azote ou sous vide. Les structures sont constituées par les substrats-supports 5 et 7, les surfaces ne devant pas être traitées, pouvant être recouvertes d'un masque. On place ensuite les structures ainsi obtenues dans un ballon contenant une solution d'un silane tel que le méthyl-n-octadécyldiéthoxysilane, à 2 % dans du toluène et on les maintient dans cette solution au reflux pendant 1 heure. On obtient ainsi une surface interne silanisée notamment sur la zone d'écoulement confiné 9. On pourrait également réaliser la silanisation sous ultrasons pendant 1 heure. Après cette opération, les structures sont rincées plusieurs fois par de l'acétone, puis séchées à 120°C. Après silanisation, on peut s'ils existent éliminer les masques.

Cette étape de silanisation permet de modifier les propriétés de surface de la silice en générant une surface hydrophobe permettant l'adsorption des anticorps.

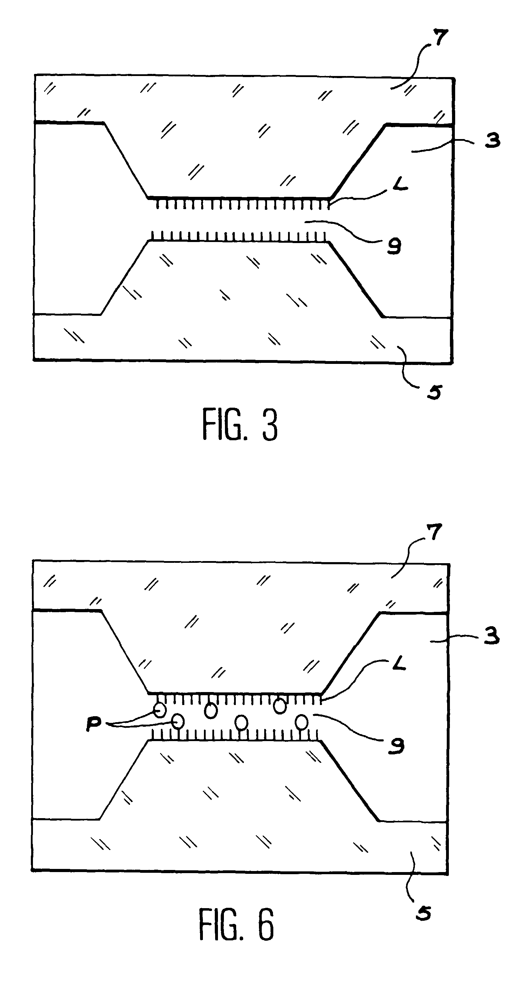

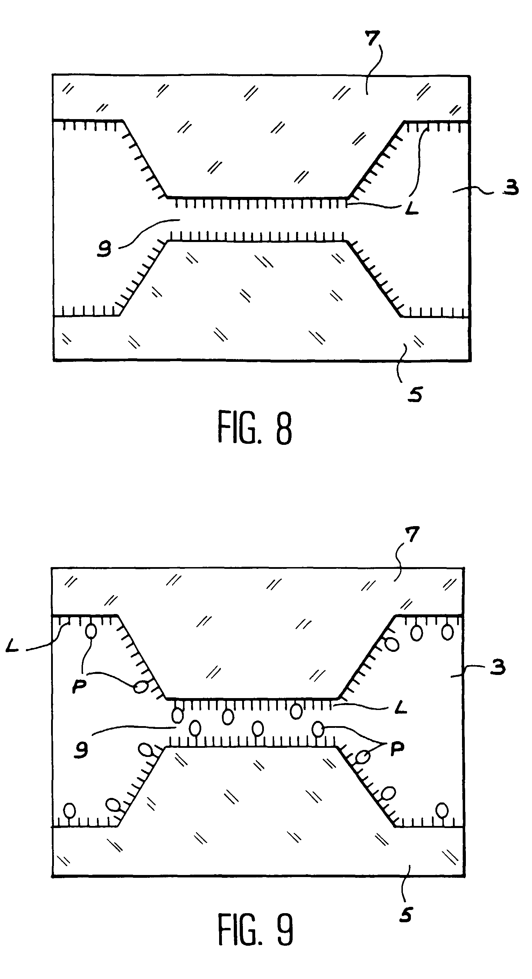

En fait, lorsque l'on utilise un masque, on obtient des surfaces traitées selon les figures 3 et 6. S'il n'y a pas de masque, ces surfaces traitées ont alors la structure représentée sur les figures 8 et 9.

Le report des substrats 5 et 7 permettant de former le conduit est réalisé soit après la silanisation, notamment pour permettre l'élimination des masques avant report lorsqu'ils existent, soit avant la silanisation.

**Exemple 2 : Fixation d'un ligand sur la paroi de la zone d'écoulement confiné.**

Dans cet exemple, le ligand est constitué par des anticorps de capture.

Pour effectuer cette fixation, on rince préalablement à l'eau l'ensemble de la cellule et des conduites, puis on les sèche à l'éthanol. On dispose dans le flacon 23 une solution d'anticorps de souris monoclonaux antiferritine à 40 µg/ml dans du tampon PBS (phosphate 50 mM, NaCl 0,15 M, pH 7,4), puis on met l'ensemble de l'installation sous vide en ouvrant les vannes A, C et D, et en fermant les vannes B et E. Puis, on rétablit brutalement la pression atmosphérique en ouvrant les vanne B, C et D et en fermant la vanne A, la vanne E restant fermée, de manière à assurer un remplissage instantané de la cellule, notamment au niveau de la zone active 9, sans formation de bulles. Cette absence de bulle est confirmée par microscopie à infrarouge permettant de visualiser l'intérieur de la zone 9. On prélève la solution d'anticorps du flacon 23 dans la seringue 17 par pompage, puis on l'injecte à l'intérieur de la cellule à un débit contrôlé au moyen du pousse-seringue 17, les vannes A, C et D étant fermées et les vannes B et E étant ouvertes. On élimine ensuite la solution d'anticorps en excès dans la seringue, et on effectue un rinçage de l'ensemble par le tampon de rinçage PBS contenant 0,5 % de Tween 20, après avoir changé le flacon 23 en réitérant les phases 3 et 4 du tableau.

Sur la figure 3, on a représenté en coupe verticale la zone active 9 de la cellule à circulation recouverte du ligand L constitué par les anticorps de capture (dans le cas particulier de l'utilisation d'un masque).

La fixation de ce ligand L est confirmée par un test enzymo-immunologique (ELISA) en utilisant des anticorps de chèvre anti-souris marqués par une enzyme : la phosphatase alcaline (AKP). Cette enzyme réagit avec son substrat spécifique, le p-nitrophénylphosphate (pNPP), pour donner un produit coloré dosé par spectrophotométrie à 405 nanomètres.

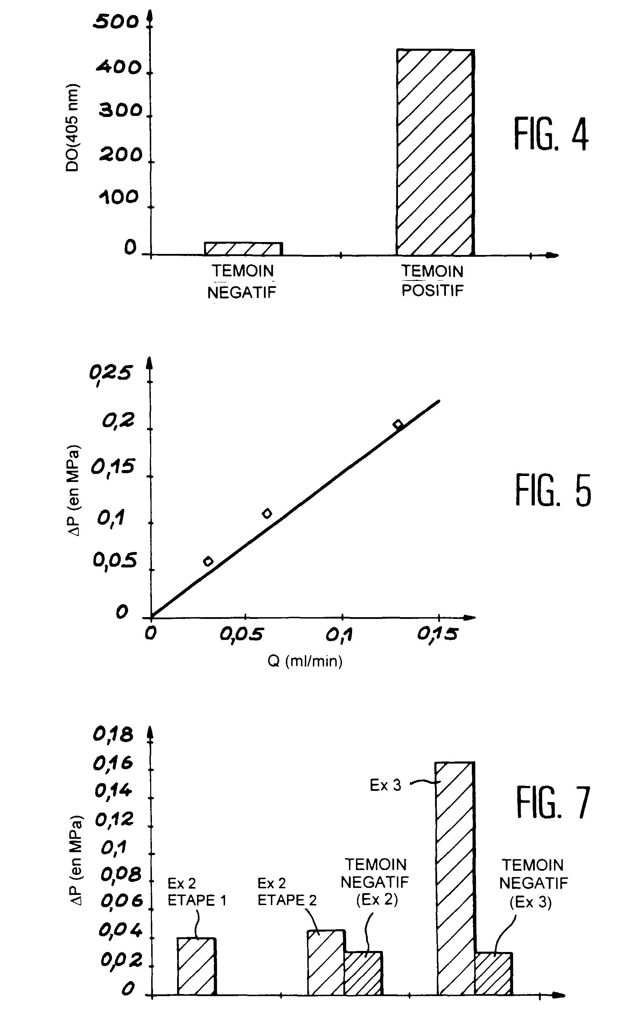

La figure 4 représente la densité optique DO à 405 nm obtenue avec la paroi revêtue des anticorps de capture (témoin positif). A titre comparatif, on a représenté également sur cette figure la densité optique obtenue avec une paroi silanisée mais qui n'a pas subi l'étape d'adsorption des anticorps de capture.

Cette figure confirme la présence des anticorps de capture dans la zone active 9 de la cellule.

On peut aussi déterminer la présence de ces anticorps de capture par le procédé de l'invention en comparant la perte de charge obtenue dans la cellule traitée par les anticorps de capture à la perte de charge obtenue dans la cellule avant traitement par la solution d'anticorps de capture.

D'après la théorie, la perte de charge $\Delta P$ lors de l'écoulement d'un fluide de viscosité dynamique µ, dans la zone d'écoulement confiné 9 de hauteur h, pour un débit Q, sur une largeur b et une longueur 1 est définie par la formule :

$$\Delta P = \frac{6\mu l}{bh^3}Q$$

Ainsi, dans le cas d'une cellule où
h = 4 µm,
b = l = 2 mm et d'un fluide dont la viscosité µ est de $10^{-3}$ kg/ms à 20°C, on a $\Delta P$ (en $10^{-1}$ MPa) = 16Q (en µl/min).

La linéarité de cette relation entre $\Delta P$ et Q dans la cellule de l'invention a été vérifié en faisant circuler le tampon PBS-Tween dans la cellule à des débits différents.

La figure 5 représente l'évolution de la perte de charge $\Delta P$ (en MPa) dans la cellule en fonction du débit Q (en ml/min) et confirme la linéarité de cette relation.

**Exemple 3 : Détermination d'un analyte constitué par des anticorps de chèvre anti-souris.**

Dans cet exemple, on utilise la cellule préparée dans l'exemple précédent, c'est-à-dire dont la paroi de la zone active 9 est revêtu d'un ligand L constitué par des anticorps de capture, anticorps de souris monoclonaux antiferritine.

Pour cette détermination, on forme tout d'abord un conjugué analyte-support pour augmenter la dimension de l'analyte. Dans ce but, on utilise comme support des particules de latex de 1 µm de diamètre et on les fait réagir avec les anticorps de chèvre anti-souris pour former le conjugué analyte-support dans du PBS Tween à une dilution de 1 : 100. Les particules de latex polystyrène-anticorps anti-souris sont commercialisées par Polyscience Inc. USA,

# EP 1 031 836 A1

sous la référence 17694.

On dispose ensuite la solution de particules de latex fonctionnalisées par les anticorps dans le flacon 23, puis on l'injecte dans la cellule comme il a été décrit précédemment dans le cas de la solution d'anticorps de capture et du tampon de lavage, à un débit de 0,3 ml/min.

On obtient ainsi la structure représentée sur la figure 6. Sur cette figure, on voit que les ligands L fixés sur la paroi de la zone active 9 de la cellule sont liés aux particules P de latex fonctionnalisées (conjugué analyte-support), qui obstruent partiellement la zone active 9 de la cellule. On fait circuler ensuite dans la cellule le tampon PBS contenant du Tween 20 à un débit de 0,3 ml/min, et on mesure la perte de charge au moyen du capteur de pression 19.

On obtient une valeur de 0,16 MPa, alors que dans le cas du témoin négatif, c'est-à-dire de la cellule ne comportant pas de ligand dans sa zone active, après injection de la solution de particules de latex fonctionnalisées, la perte de charge est de 0,03 MPa. Ceci s'explique par l'impossibilité de fixer le conjugué particules de latex-anticorps anti-souris sur la paroi de la zone active de la cellule qui ne comporte pas le ligand formé par les anticorps de capture.

Sur la figure 7, on a représenté les pertes de charge dans le cas des exemples 2 (étapes 1 et 2) et 3 et celles des témoins négatifs (pas d'anticorps de capture) dans ces deux exemples.

L'étape 1 correspond à la perte de charge mesurée en amont de la cellule lors de l'injection des anticorps. L'étape 2 correspond à la mesure avec le liquide de lavage PBS/tween 20 après la fixation des anticorps (débit de 0,03 ml/min). Dans l'exemple 3, la mesure est effectuée avec avec du PBS-Tween 20 à un débit de 0,03 ml/min.

Dans le cas de l'exemple 2 (étapes 1 et 2), on remarque une légère augmentation de la perte de charge due à la fixation des anticorps de capture. Dans le cas de l'exemple 3, l'augmentation de perte de charge est importante. Par ailleurs, on note d'après les résultats des étapes 1 et 2, qu'il n'y a pas d'augmentation de perte de charge lorsque l'on change de solution.

On constate ainsi que l'immobilisation spécifique des conjugués anticorps-particules de latex à l'intérieur de la cellule à circulation provoque une diminution de la hauteur de la zone active 9, ce qui se traduit par une augmentation de la perte de charge à débit constant.

Dans le cas du témoin négatif de l'exemple 3, aucune variation de perte de charge n'est observée au cours de l'étape de rinçage de l'exemple 3. Les particules de latex n'ont pas modifié la hauteur de la zone active 9 car elles ne sont pas retenues dans cette zone active.

Les figures 8 et 9 illustrent des cellules dans lesquelles toute la surface du conduit de circulation 3 est revêtue de ligand L (figure 8), puis de particules P de conjugué analyte-support (figure 9). Ceci correspond au cas où toute la surface interne du conduit est fonctionnalisée, comme dans l'exemple 1 sans utiliser de masque, et où l'on procède ensuite à la fixation du ligand comme dans l'exemple 2, puis à la détermination de l'analyte comme dans l'exemple 3.

Ainsi, on trouve les particules P dans le conduit 3 et la zone confinée 9. Cette présence ne peut influer sur la perte de charge qui sera mesurée, et constitue un mode préférentiel de réalisation car il est beaucoup plus simple à mettre en oeuvre.

## REFERENCES CITEES

[1] : FR-A-2 758 884.

[2] : US-A-4 964 847.

[3] : FR-A-2 727 648.

## Revendications

**1.** Procédé de détermination d'un analyte dans une solution, comprenant les étapes suivantes :

   a) fixer et immobiliser ledit analyte sur la surface interne d'un conduit ayant une section transversale réduite sur tout ou partie de celui-ci, et
   b) déterminer la variation de la perte de charge d'un fluide circulant dans le conduit, due à l'analyte qui a été fixé et immobilisé au moins dans la section transversale réduite dudit conduit lors de l'étape a).

**2.** Procédé selon la revendication 1, dans lequel l'étape a) est effectuée en mettant en contact la solution avec la surface interne du conduit, ou en faisant circuler la solution dans le conduit, cette circulation pouvant comporter au moins un arrêt séquentiel.

**3.** Procédé selon la revendication 1, dans lequel on détermine la variation de la perte de charge en mesurant la différence de pression à débit constant entre deux points situés de part et d'autre de la section transversale réduite du conduit, d'un fluide circulant dans le conduit, avant de réaliser l'étape a) et après avoir réalisé l'étape a) du procédé, ou la différence de débit à pression constante avant et après l'étape a).

4. Procédé selon la revendication 1 ou 3, dans lequel ledit fluide est toute ou partie de la solution.

5. Procédé selon la revendication 1 ou 3, dans lequel ledit fluide est un liquide différent de la solution.

6. Procédé selon l'une quelconque des revendications 1 à 5, dans lequel préalablement à l'étape a), la paroi de la section transversale réduite est revêtue sur tout ou partie d'au moins un ligand capable de se fixer à l'analyte.

7. Procédé selon la revendication 6, dans lequel on détecte la présence du ligand en déterminant la variation de la perte de charge en mesurant la différence de pression à débit constant ou la différence de débit à pression constante entre deux points situés de part et d'autre de la section transversale réduite du conduit, d'un liquide circulant dans le conduit, avant et après avoir réalisé le revêtement de ligand.

8. Procédé selon l'une quelconque des revendications 1 à 7, qui comprend de plus une réaction physique ou chimique de l'analyte avec un matériau-support pour former un conjugué analyte-support de dimension plus importante que celle de l'analyte, cette réaction intervenant soit lorsque ledit analyte est libre dans la solution, avant l'étape a), soit lorsque l'analyte est déjà fixé directement ou indirectement sur la paroi interne de la section réduite du conduit après l'étape a) pour constituer un conjugué analyte-support.

9. Procédé selon la revendication 8, dans lequel le matériau-support comprend un ligand capable de se lier à l'analyte.

10. Procédé selon la revendication 8 ou 9, dans lequel le matériau-support est constitué de particules de latex fonctionnalisées par un ligand spécifique de l'analyte.

11. Procédé selon l'une quelconque des revendications 1 à 10, qui comprend au moins une étape de lavage du conduit entre les étapes a) et b).

12. Procédé selon l'une quelconque des revendications 1 à 11, dans lequel la plus petite dimension $T_c$ de la section transversale réduite du conduit est telle que :

$$(T_c : 3) < T_A \text{ ou } e < T_c$$

avec $T_A$ représentant la dimension de l'analyte fixé tel quel ou sous la forme d'un conjugué analyte-support, ou e représentant l'épaisseur du ligand.

FIG. 1

FIG. 2

EP 1 031 836 A1

FIG. 3

FIG. 6

FIG. 4

FIG. 5

FIG. 7

EP 1 031 836 A1

FIG. 8

FIG. 9

14

**EP 1 031 836 A1**

Office européen
des brevets

**RAPPORT DE RECHERCHE EUROPEENNE**

Numéro de la demande

EP 00 40 0449

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | FR 2 758 884 A (BIO MERIEUX)<br>31 juillet 1998 (1998-07-31)<br>* le document en entier *<br>--- | 1 | G01N33/543 |
| D,A | US 4 964 847 A (PRINCE PAUL R)<br>23 octobre 1990 (1990-10-23)<br>* le document en entier *<br>----- | 1 | |

DOMAINES TECHNIQUES
RECHERCHES (Int.Cl.7)

G01N

Le présent rapport a été établi pour toutes les revendications

| Lieu de la recherche | Date d'achèvement de la recherche | Examinateur |
|---|---|---|
| LA HAYE | 10 avril 2000 | Moreno, C |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

EPO FORM 1503 03.82 (P04C02)

15

**ANNEXE AU RAPPORT DE RECHERCHE EUROPEENNE
RELATIF A LA DEMANDE DE BREVET EUROPEEN NO.**

EP 00 40 0449

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

10-04-2000

| Document brevet cité au rapport de recherche | | Date de publication | Membre(s) de la famille de brevet(s) | | Date de publication |
|---|---|---|---|---|---|
| FR 2758884 | A | 31-07-1998 | AU | 6104598 A | 25-08-1998 |
| | | | EP | 0965045 A | 22-12-1999 |
| | | | WO | 9834116 A | 06-08-1998 |
| US 4964847 | A | 23-10-1990 | AU | 605206 B | 10-01-1991 |
| | | | AU | 8172887 A | 06-05-1988 |
| | | | CA | 1308007 A | 29-09-1992 |
| | | | DE | 3781677 A | 15-10-1992 |
| | | | EP | 0287651 A | 26-10-1988 |
| | | | JP | 2553899 B | 13-11-1996 |
| | | | JP | 1501170 T | 20-04-1989 |
| | | | WO | 8802864 A | 21-04-1988 |

EPO FORM P0460

Pour tout renseignement concernant cette annexe : voir Journal Officiel de l'Office européen des brevets, No.12/82